# EUROPEAN PATENT SPECIFICATION

(11) **EP 0 535 612 B1**
(45) Date of publication and mention of the grant of the patent: **17.06.1998**
(21) Application number: 92116682.3
(22) Date of filing: 29.09.1992
(51) Int. Cl.: G01N 35/10, A61L 2/20

(54) **Method for regenerating biologically used devices**
Verfahren zum Regenerieren von zur Handhabung von biologischen Substanzen verwendeten Geräten
Procédé de régéneration de dispositifs pour la manutention de substances biologiques

(30) Priority: 30.09.1991 JP 251903/91; 28.02.1992 JP 43774/92
(43) Date of publication of application: 07.04.1993
(73) Proprietor: OLYMPUS OPTICAL CO., LTD., Tokyo 151 (JP)
(72) Inventor: Nakamura, Makoto, c/o Intel. Prop. & Legal Dep., Hachioji-shi, Tokyo (JP); Kawakami, Shinsuke, c/o Intel. Prop. & Legal Dep., Hachioji-shi, Tokyo (JP); Ichinohe, Masafumi, c/o Intel. Prop. & Legal Dep., Hachioji-shi, Tokyo (JP); Kaneko, Yasunobu, c/o Intel. Prop. & Legal Dep., Hachioji-shi, Tokyo (JP)
(74) Representative: KUHNEN, WACKER & PARTNER

(56) References cited:
- WO-A-83/00447
- DD-A- 267 114
- DE-A- 1 951 511
- US-A- 3 751 225
- US-A- 4 517 159
- US-A- 4 989 623
- PATENT ABSTRACTS OF JAPAN vol. 15, no. 221 (C-838)6 June 1991
- PAJP Vol 15,No 302(C0855) & JP-A-3-111 026

## Description

The present invention relates to a cleaning method of devices for handling or treating biologically active substances or samples containing such the active substances. More particularly, the invention relates to a method for cleaning the used devices such as sample nozzles, reagent dispensing nozzles, reaction vessels or the like employed in analyzing apparatus which have been contacted with the samples or reagents, in order to regenerate the device for re-use.

The invention also relates to analyzing apparatus and dispensing apparatus having such devices, and more particularly to a cleaning system for cleaning these devices to be in a re-usable state.

In clinical laboratory tests, by using various analyzing apparatuses, compositions of various body fluids or humors such as urine, serum and plasma are analyzed. In such analyses, not limited to the ordinary biochemical measurement methods, various measurement methods such as the immunological measurement method using antigen-antibody reaction, the measurement method using DNA probe, the measurement method using electrophoresis are employed.

In such measurement methods, in order to obtain an accurate result of measurement, it is required that the devices to be used be clean. Above all, as the method of analysis is becoming higher in sensitivity and the measuring range is extended, cleaning of the devices such as sample dispensing nozzle and reaction vessels which contact with the samples is becoming more and more important recently.

Hitherto, many methods have been proposed for cleaning the nozzles and containers used in analyses. Basically, most of them are intended to enhance the cleaning effect mechanically by using cleaning water or detergent (Unexamined Japanese Patent Publication 62-242858, 62-288574, Examined Japanese Utility Model Publication 3-23572, Examined Japanese Patent Publication 52-28754). For example, the cleaning mechanism disclosed in 62-288574 is designed to blow the cleaning water from below to above, move a sampling probe which is used for sucking and discharging the sample and reagent, and to clean the outer wall of the sampling probe by the cleaning water when its end passes through the blowing part of the cleaning water.

However, there is a limit for attainable cleaness if the cleaning effect is merely enhanced mechanically, and it is not sufficient. In particular, in the recent analysis of advanced high sensitivity, even when the cleaning with water is employed, the carry-over and contamination cannot be completely avoided at the practical level. In addition, if it is attempted to avoid carry-over or contamination by using mechanical cleaning with water, a large volume of cleaning water is needed, and the amount of waste liquid increases, which results in higher treatment cost. Furthermore, an extra time is needed for washing away the cleaning chemical, and it is hence not suitable for mass treatment or continuous treatment. Accordingly, so far, multiple dispensing nozzles are used, or the reaction line is extended in order to obtain a longer available cleaning time.

On the other hand, in the Unexamined Japanese Patent Publication 1-254871, a cleaning method using a piezoelectric vibrator is proposed. In this method, although the cleaning efficiency is expected to be improved, the substance adsorbed on the nozzle keeps the absorbance equilibrium on the nozzle surface.
Therefore, it may not be always an effective cleaning method depending on the strength of affinity between the adsorbed substance and the probe surface.

Accordingly, there has been proposed the mechanism for making the parts such as sample nozzle which directly contact with the sample to be disposable, (Unexamined Japanese Patent Publication 63-243762, 1-184465, Unexamined Japanese Utility Model Publication 1-146162, 1-141466, Unexamined Japanese Patent Publication 63-106567).

If the parts or devices are constructed to be disposable, the problems of contamination and carry-over between samples due to improper cleaning of sample nozzles may be avoided securely. However, discarding the sample nozzle after measurement of every sample not only increases the cost, but also poses problems against the environmental protection highly demanded recently.

Furthermore, US-A-4 517 159 describes a method for sterilization for hospital and field use in which an article to be sterilized is submerged in water and ozone.

US-A-4 989 623 discloses a cleaning apparatus for cleaning delivery probes used for dispensing protein-containing reagents.

Thus, the prior arts have their own merits and demerits, and it is difficult to satisfy all requirements of non-disposable, non-carrry-over, and non-contamination. In such situation, technical development concerning the prevention of contamination or carry-over is inclined toward the disposable trend as far as reviewed from the recent patent applications. It means how difficult it is to develop a perfect cleaning method.

It is hence a first object of the invention to challenge the difficulty mentioned above and to provide an effective cleaning method not found in the past in any sort, which is capable of decreasing the waste liquid output by setting in a nearly non-cleaning state as seen from the conventional concept of cleaning, reducing the size of the waste liquid tank, and re-using the device practically without disposing the same.

This object is attained by a method according to claim 1.

The first object is achieved by a method of regenerating a biologically used device in order to re-use the device for next treatment of a biologically active substance or a sample containing the substance, the method comprising a step of inactivating the active substance remaining on a surface of the device while leaving the substance on the surface, thereby depriving the remaining substance of a characteristic which adversely affect the next treatment of the active substance or the sample.

This invention can be more fully understood from the following detailed description when taken in conjunction with the accompanying drawings, in which:

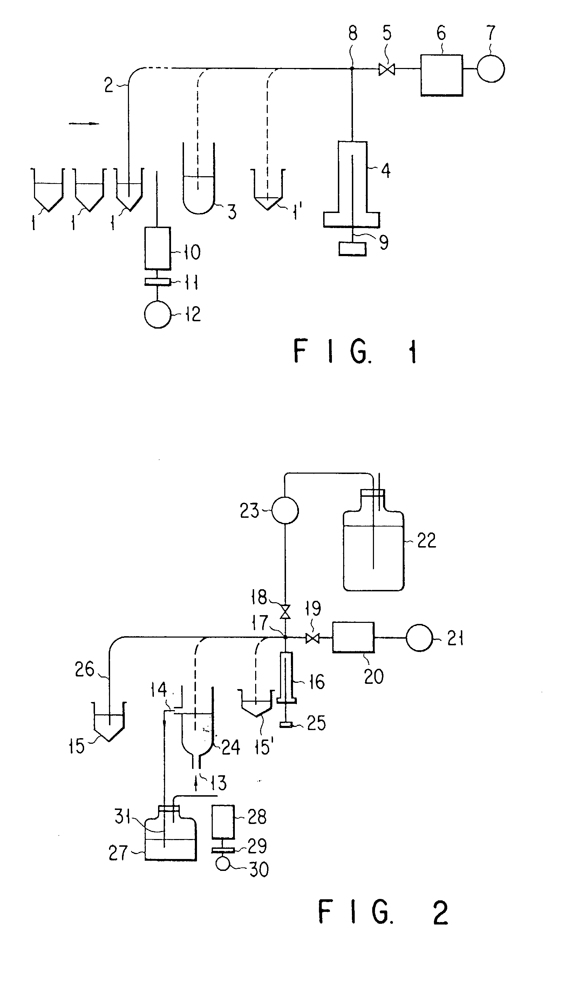

Fig. 1 through Fig. 11 are schematic views showing embodiments of the cleaning method and cleaning system of the present invention.

The invention is described in detail below.

It is mainly in the case of immunological measurement or biological activity measurement such as DNA assay using DNA probe that the complete degree of cleaning is required in the device or parts contacting with the samples or reagents, such as sample nozzles, reagent dispensing nozzles, and reaction vessels used in the analyzer. All these measurements are extremely advanced in recent years with respect to the measuring range and sensitivity. In addition, when observing the cells or the like histochemically by staining or luminescence, the biological activities of antigen, antibody and enzyme are often utilized, and hence contamination with these substances from other cells must be absolutely avoided in the process of preparing segments or the like of specimens.

In such a case, it is not the only means for effective cleaning to merely wash away completely the biologically active substances such as antigen, antibody, enzyme, bacteria, virus, DNA, RNA which are contained in and given from samples or reagents. That is, the purpose of cleaning is to regenerate the devices so as to be re-usable, and when the active substances existing in the samples or reagents deposited on the surface of the device are inactivated or made inert, the purpose of the cleaning is effectively achieved.

On the basis of this basic concept, the present inventors investigated the effects of various inactivation treatments for regenerating the devices having been previously used. In particular, studies were made with respect to the method capable of denaturating or decomposing protein.

In the past, the protein denaturing agent and protein inactivating agent were used as sterilizer and disinfectant, which may include, for example, the following: acids or alkalis (e.g. mineral acid, caustic soda, barium hydroxide, benzoic acid, parahydroxy benzoic acid, lactic acid, propionic acid), heavy metals (e.g. corrosive sublimate, Mercurochrome, Merthiolate, mercury phenyl acetate or other mercury compound, Salvarsan or other arsenic compound, copper sulfate, silver nitride), oxidizing agents (e.g. potassium permanganate, bromine, iodine, fluorine or other halogens), formalin, and dye agent (e.g. basic or acidic dye).

However, the conventional protein denaturation or protein inactivation caused by using chemical reagent, ultraviolet rays, heated steam, ultrasonic waves or the like for the purpose of sterilization or disinfection was intended to suppress, weaken or destroy the harmful vital activities of microorganisms such as infection, parasitism, and proliferation, which is not always same as elimination of activity of substance such as antigen, antibody and enzyme. This is evident from the fact that the antigen and antibody are generally utilized with their activity being still effective, after being subjected to treatment similar to sterilization or disinfection, such as refining of proteins by ethanol or acetone, aseptic treatment of insoluble antigen by using formalin or phenol, and immunohistochemical staining after fixing with formalin. In other words, sterilization or disinfection is intended to pretreat a sample containing the boilogically active substance to be treated in order to destroy the microorganism as foreign matter other than the objective substance, and therefor, it should not be the treatment for inactivating the biologically active substance.

It was brought into consideration and studied to use the conventional protein denaturing agents and inactivating agents by adding to cleaning solution. But the remained denaturing agents adversely influenced the results of analysis or caused chemical damages to the analyzing apparatus. Since the cleaning time in a general analyzing apparatus is only several seconds, the concentration of the degenerating or inactivating agent must be raised in order to have a sufficient effect, which was not considered practicable. This is just as expected, and it seems because of this fact that effective cleaning means based on the protein denaturing agent is not realized yet in spite of investigations by many engineers.

Under the circumstances, the present inventors took notice of peroxide gas. Generally, peroxide gases such as ozone and hydrogen peroxide possess a potent oxidizing action, and are unstable and likely to be converted inert, and therefore the residual effects is nearly zero. This nature is important and advantageous for realizing the basic concept of the invention in the sense that no adverse effects are given to the result of analysis. In particular, the amount and time of ozone generation can be electrically controlled, which is an important nature not found in other substances.

It is known that cystein, methionine, cystin, tyrosine, and histidine are affected by being subjected to the powerful oxidizing action of ozone, (M. Kuroda, F. Sakiyama, K. Narita, J. Biochem., 78, 641, 1975). It has been also proposed to use ozone in sterilization and disinfection (Unexamined Japanese Patent Publication 63-236593, 61-138586, 61-263691). Furthermore, an apparatus for sterilizing endoscope by using ozone water is proposed (Unexamined Japanese Patent Publication 3-111026, 3-68331).

Actually, however, since the time required for sterilization or disinfection varies significantly depending on the difference in the size, morphology, tolerance and protein composition of microorganisms, and since the rate of sterilizing action decreases as approaching aseptic state, the treatment is usually carried out in a range of 1 to 24 hours. Therefore, there is no such a suggestion in the prior arts that the effect of completing sterilization or disinfection is expected from ozone treatment for about several seconds to about a minute, or in a short time available in cleaning of the analyzing apparatus. In addition, there is no report suggesting possibility of eliminating the activity of antigen, antibody, enzyme or DNA at practical level. To the contrary, it was estimated in a certain report that denaturation of protein required scores of minutes (Utility Water and Waste Water, p. 894, vol. 30, No. 9, 1988). Generally considering the teachings and report described in the published papers, the realization of the basic concept of the invention seemed to be impossible by using ozone.

As a result of intensive studies under such circumstances, the inventors confirmed that ozone water, water mixed with ozone-containing air foams, or ozone-containing air could be effectively used in cleaning of analyzing apparatus. In particular, cleaning with ozone-containing air was effective, and brought about results not found in the past. This result was quite unexpected and was surprising. Accordingly, by combining the astonishing result with the known advantage that the amount and the time of ozone generation can be electrically controlled, the invention has been completed, making it possible to provide an optimum cleaning method for analyzing apparatus and a cleaning system.

Devices to which the method of the invention may be applied include dispensing nozzles, syringes, suction tubes, containers for various samples and reagents, reaction vessels, measuring vessels, agitators, filters, sensors, and others. In the case of applying the present invention to the analyzing apparatus, cleaning related parts such as the device-exchanging system, waste liquid system may be extremely decreased or completely omitted.

Moreover, the invention also presents a recycling technology for re-using the cleaning water after use, in the cleaning system which comprises precleaning system employing cleaning water not containing cleaning chemicals. Examples of cleaning water used in precleaning include purified water, physiological saline, buffer solution, and dilution solution, which may be used only for washing away the sample deposits from the surface of the device.

The cleaning method and system of the invention may be applied to the apparatus employing various methods of analysis. In particular, the invention is applied to the apparatus for immunological measurement, for example, the analyzing apparatus for coagulation reaction of particles such as erythrocytes, latex immunoassay, immune specific turbidity radioimmunoassay, enzyme immunoassay, fluoroimmunoassy, or chemical luminescence immunoassay. Furthermore, in the applications where DNA, RNA and others are used as samples, or DNA probe is used as reagent, the oxidizing action of ozone is expected to present a sufficient effect. The invention is not limited to the particular type of specimen, and may be applied, for example, in humors or body fluids such as blood, lymph and urine or stool. In particular, in the analysis using urine or stool, the deodorizing effect of waste liquid is also expected.

Handling of peroxide gas such as ozone may be carried out according to the conditions disclosed in the literature or handbook, and it may be also preferred to install any means for recovering or decomposing the ozone after use as required.

The regeneration method of the invention is based on the inactivation of the biological activity common to the living body, such as the activity of antigen-antibody reaction and enzymatic reaction, and therefore fluctuations are very small in the effects depending on the type of microorganism, as experienced in sterilization or disinfection mentioned previously. In addition, since the cleaning time is very short, it may be flexibly applied to any analyzing apparatus.

The cleaning system of the invention is a realization of the basic concept as an apparatus. That is, when using ozone as the inactivating fluid, the present invention comprises an ozone generator, a mechanism for causing the ozone-containing gas or fluid to be contacted with the sample nozzle, reagent dispensing nozzle, reaction vessel, or other parts which contact with sample or reagent, and a mechanism for recovering and decomposing the ozone after treatment. In this case, the ozone generator to be used may be a commercial one. The mechanism for causing the ozone-containg gas or fluid to be contacted with the device parts to be cleaned may include, for example, the following system.
· Passing ozone-containing gas through the sample nozzle.
· Passing ozone-containing fluid through the sample nozzle.
· Cleaning by forming fine foams of ozone-containing air in water.
· Blowing or sucking ozone-containing air into the sample nozzle.
· Retaining reaction vessels in an ozone treatment compartment.

Furthermore, as the physicochemical technology for recovering and decomposing the wasted ozone to make it harmless, those mentioned in the published papers or handbooks may be employed.

In the execution of the invention, in the first place, the fluid for inactivation, for example, ozone is generated by an ozone generator. The ozone-containing air is, either in gas form or in a state dissolved in liquid, brought into contact with the used sample nozzle or reagent dispensing nozzle, or reaction vessel or reagent storage container or the like, thereby inactivating the biologically active substances such as antigen, antibody and enzyme. The ozone-containing gas or fluid after the treatment is recovered, decomposed or otherwise processed to make it harmless.

In this way, the devices for treating biologically active substances or samples containing such active substances are cleaned to a practically re-usable level in several seconds to a minute. Therefore, when the method of the invention having such performance is applied to the apparatus of the invention, it is possible to sufficiently cope with the continuous handling of samples or the like. That is, according to the cleaning mechanism of the invention, the dispensing nozzles may be promptly cleaned while continuously dispensing similar or different samples. At this time, when the apparatus is composed to use plural nozzles alternately, a faster continuous dispensing and inactivation treatment is realized. If necessary, after every predetermined number of times of dispensing, inactivation treatment for about a minute may be done. Or in cleaning of reaction vessels, since it is possible to re-use promptly, the reaction line may be notably shortened.

Referring now to examples, the invention is described in further details below, but these examples are not intended to limit the scope of the invention.

### Example 1

This embodiment relates to a cleaning method by using ozone-containing air, and a dispensing machine having a cleaning system for executing such cleaning method.

Fig. 1 is an example of an apparatus according to the present embodiment. This apparatus is composed so as to clean by passing ozone-containing air. To be precise, it is intended to inactivate the boilogically active components in the sample, rather than to clean.

In Fig. 1, numeral 1 denotes a sample cup containing sample, 1' is a reaction vessel, 2 is a nozzle for dispensing the sample into the reaction vessel 1', 3 is a vessel for cleaning the nozzle, 4 is a syringe, 5 is an electromagnetic valve, 6 is an ozone generator, 7 is an air pump, 8 is a coupling unit, 9 is a plunger, 10 is an ozone processing device, 11 is a filter, and 12 is a suction pump.

In the embodiment using this apparatus, the sample in the sample cup 1 is sucked by the nozzle 2 by the action of the plunger 9, and is discharged into the reaction vessel 1', and the nozzle 2 is cleaned in the container 3 by the ozone-containing air. The steps of the cleaning process are extremely few, and only the air is sent into the ozone generator 6 by the air pump, and the ozone-containing air is released from the nozzle 2 for a proper time. Releasing pressure of the ozone-containing gas is suitably selected depending on the nozzle shape and the nature of the sample. At this time, the container 3 may not be always needed, but the ozone-containing air is more likely to contact with the inner wall and outer wall of the nozzle 2 in the container 3, and it is more effective to use the container 3. Cleaning by the gas also involves the water draining effect and clogging preventive effect. Or liquid such as water may be contained in the container 3, and the nozzle 2 may be bubbled in the water by the ozone-containing air. This liquid is, however, not always necessary.

Furthermore, after releasing the ozone-containing air from the nozzle 2 for a proper time, the ozone generator 6 is stopped, and only the air may be sent into the nozzle 2 by the suction and discharge operation of the air pump 7 or syringe 4. When the ozone-containing air adversely affects the analysis, it is desired to prevent the effect of ozone in this manner.

The ozone-containing air is sucked by the suction pump 12, and is discharged through the ozone processing device 10 and filter 11. The filter 11 is not absolutely necessary.

The cleaning process by using the apparatus in Fig. 1 is explained in detail below. First, the nozzle 2 is moved into the sample cup 1 containing the sample, and the front end of the nozzle 2 is dipped in the sample, and the sample is sucked by pulling the plunger 9. Afterwards, the nozzle 2 is moved to the reaction vessel 1', and the plunger 9 is pushed to discharge the sample. Consequently, the nozzle 2 moves to the cleaning vessel 3, and the electromagnetic valve 5 (which is normally closed) is opened, and at the same time the switch of the ozone generator 6 is turned on, and the air pump 7 forces out the air so that the ozone-containing air comes out of the nozzle 2. When cleaning is over, the electromagnetic valve 5 is closed, and the nozzle 2 moves to another sample cup, thereby sucking the sample. The same operation is thereafter repeated.

The cleaning method in this example may be also executed by employing the apparatus in Fig. 2. In Fig. 2, numeral 13 is a water feed port, 14 is a discharge port, 15 is a sample cup, 15' is a reaction vessel, 16 is a syringe, 17 is a coupling part, 18, 19 are electromagnetic valves, 20 is an ozone generator, 21 is an air pump, 22 is a water tank, 23 is a feed water pump, 24 is a vessel for cleaning the nozzle, 25 is a plunger, 26 is a nozzle, 27 is a waste liquid tank, 28 is an ozone processing device, 29 is a filter, 30 is a suction pump, and 31 is a waste liquid pipe.

The structural feature in Fig. 2 is that, as compared with the apparatus in Fig. 1, mechanical cleaning is also effected by the solution supplied from the feed water tank 22. The container 24 for cleaning the nozzle may receive the solution from the feed water port 13, or only operation of water discharge from discharge port 14 may be done. The nozzle 26 sucks the sample in the sample cup 15, discharges into the specified reaction vessel 15', and is cleaned in the container 24. More preferably, after once cleaning the nozzle 26 with the solution supplied from the feed water tank 22, it is desired to clean the nozzle 26 with the ozone-containing air as in the case of Fig. 1.

The waste liquid and ozone-containing air are sucked by the suction pump 30, and the waste liquid is collected in the waste liquid tank 27, while the ozone-containing air is discharged through the ozone processing device 28 and filter 29.

The solution collected in the waste liquid tank 27 is inactivated by being bubbled or agitated with the ozone-containing air discharged together with the waste liquid through the waste liquid pipe 31 extended from the discharge port 14. By the bubbling or agitation, disinfection of the waste liquid can be also achieved. At this time, by commonly using the feed water tank 22 and waste liquid tank 27 or coupling them together, the waste liquid thus inactivated may be recycled for re-using as the cleaning solution so that the trouble of discarding the waste liquid may be excluded.

Explaining the cleaning process employing the apparatus in Fig. 2 in further detail as follows. After the nozzle 26 is moved to the cleaning container 24, the electromagnetic valve 18 is opened, and the water is supplied from the tank 22 by the feed water pump 23, and is discharged from the nozzle 26. Subsequently, the electromagnetic valve 18 is closed, and the electromagnetic valve 19 is opened. At the same time, the power source of the ozone generator 20 is turned on, and the air is forced out by the air pump 21. As a result, the ozone-containing air comes out of the nozzle 26. After cleaning, the electromagnetic valve 19 is closed, and the ozone generator stops. Then the nozzle 26 moves to the next sample cup, and the sample is sucked and the same operation is repeated.

The cleaning experiment using the apparatus in Fig. 1 is explained below.

### <Measurement of cleaning effect (1)>

After sucking 25 µL (L denotes the liter here and hereinafter) of anti-HBs antibody solution through the nozzle 2, it was discharged. Then, the nozzle 2 was cleaned according to the method described in Fig. 1. The antibody titer of the anti-HBs antibody was 2¹⁸. The titer was measured with anti-HBs antibody measuring reagent which is available from Chemical and Serum Therapy Research Institute under the trade name of Serocrit anti-HBs. The measuring conditions were as follows.
· Liquid volume of vessel 3: purified water 7 ml
· Ozone output of ozone generator 6: 50 mg/h
· Air feed rate of air pump 7: 1.2 L/min
· Cleaning time: 5 sec

Meanwhile, the nozzle 2 was bubbled in the vessel 3. As the control, cleaning was also conducted in the condition of ozone output of 0 mg/h.

In this way, the operation from suction of anti-HBs antibody solution to cleaning was repeated three times in the same vessel 3. At this time, together with the cleaning effect of the nozzle 2, the increase of contamination in the vessel 3 was also investigated.

After cleaning the nozzle 2 under the above conditions, the presence or absence of the anti-HBs antibody left over in the nozzle 2 was investigated. The measuring operation was as follows.

First, 50 µL of specimen dilution solution of anti-HBs antibody measuring reagent (Serocrit anti-HBs) was sucked through the nozzle 2 after cleaning, and discharged. To 25 µL of this discharged solution, 25 µL of HBs Ag sensitized blood cells of Serocrit anti-HBs was added to react in a V-bottom microplate. At this time, depending on the presence or absence of coagulation of HBs Ag sensitized blood cells, the presence or absence of anti-HBs antigen remaining in the nozzle 2 was investigated. The result of measurement was as shown in Table 1. The criterion in the table conforms to the instructions in the package insert of the reagent. In other words, (++) and (+) means the presence of contamination, (-) means free from contamination, and (±) denotes a slight contamination but is practically free from effects on measurement.

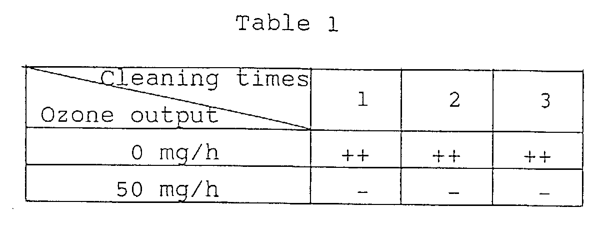

Estimating based on the changes from (++) at ozone output of 0 mg/h to (-) at ozone output of 50 mg/h shown in Table 1, only by feeding the ozone-containing air into the nozzle 2, decrease of antibody titer of 2¹⁸ or more was confirmed. This result proves a very high cleaning effect of this method. In addition, although only 7 ml of purified water is present in the container 3, a sufficient cleaning effect is maintained even when the cleaning is repeated in the same vessel 3, which is an effect not found in the conventional cleaning methods.

### <Measurement of cleaning effect (2)>

Cleaning effect was measured as same as in the measurement of cleaning effect (1), except that the sample was changed from anti-HBs antibody solution to HBs Ag solution, and the effect was measured. The titer of HBs Ag was 2¹⁷ which was measured with HBs antigen measuring reagent (Serodia HBs) available from Fuji Rebio. In the measurement, HBs antigen measuring regenerate (Serodia HBs) was used. The result of measurement was as shown in Table 2.

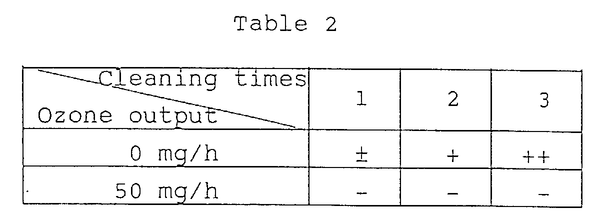

As shown in Table 2, while a very high cleaning effect is proved only by feeding ozone-containg air into the nozzle 2, HBs Ag is left in the nozzle 2 in the case of air alone, the results being very reasonable. As cleaning was repeated, the HBs Ag amount in the vessel 3 increased consequently, it is found that HBs Ag becomes more likely to be left over in the nozzle 2 by repeating the cleaning.

### Example 2

This embodiment relates to an apparatus designed to clean by generating fine foams of ozone-containing air in water.

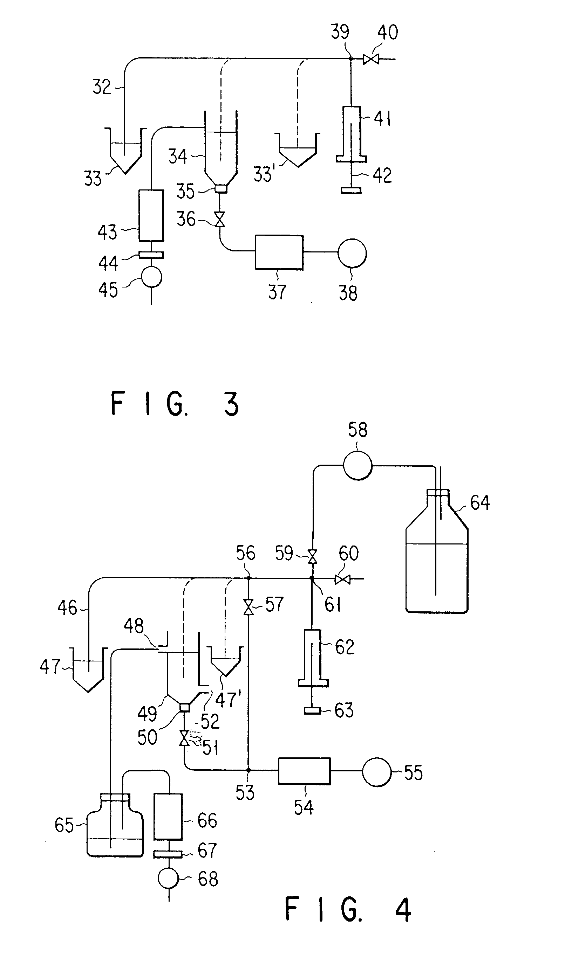

Fig. 3 shows the apparatus used in this embodiment. In the drawing, numeral 32 denotes a sample nozzle, 33 is a sample cup, 34 is a cleaning vessel, 33' is a reaction vessel, 35 is a foam generator, 36 is an electromagnetic valve, 37 is an ozone generator, 38 is an air pump, 39 is a coupling part, 40 is an electromagnetic valve, 41 is a syringe, 42 is a plunger, 43 is an ozone processing device, 44 is a filter, and 45 is a suction pump.

In cleaning by using this apparatus, air is sent into the ozonizer 37 by the air pump 38, and the ozone-containing air is supplied into the cleaning vessel 34 through the foam generator 35. The foam generator 35 may be a porous filter or ultrasonic vibrator, or the like. The inside of the nozzle 32 is cleaned by sucking and discharging, by the plunger 42, the solution in the cleaning vessel 34 in which the foams of ozone-containing air being generated.

### <Measurement of cleaning effect>

After sucking and discharging 25 µL of two types of anti-HBs antibody solution, the nozzle 24 was cleaned by using the apparatus shown in Fig. 3. The antibody titers of the two types of the anti-HBs antibody solution were respectively 2¹⁵ and 2¹⁸. The titers are measured with anti-HBs antibody measuring reagent which is available from Chemical and Serum Therapy Research Institute under the trade name of Serocrit anti-HBs. The conditions of cleaning were as follows.
· Solution in vessel 34: Purified water 10 ml
· Ozone output by ozone generator 37: 50 mg/h
· Air feed rate of air pump 38: 1.2 L/min
· Cleaning by suction and discharge: 5 sec, twice

As the control, cleaning was also effected in the condition of ozone output of 0 mg/h.

This operation from suction of anti-HBs antibody solution till cleaning was repeated three times in the same vessel 34. At this time, together with the cleaning effect of the nozzle 32, increase of contamination in the vessel 34 was also investigated.

After cleaning the nozzle 32 in the above condition, the presence or absence of the anti-HBs antibody left over in the nozzle 32 was investigated. The measuring operation was as follows.

First, 50 µL of specimen dilution solution of anti-HBs antibody measuring reagent (Serocrit anti-HBs) was sucked through the nozzle 32 after cleaning, and discharged. To 25 µL of the discharge solution, 25 µL of HBs Ag sensitized blood cells of Serocrit anti-HBs was added to react in a V-bottom microplate. At this time, based on the presence or absence of coagulation of HBs Ag sensitized blood cells, the presence or absence of anti-HBs antibody remained in the nozzle 32 was investigated. The results of measurements were as shown in Tables 3, 4.

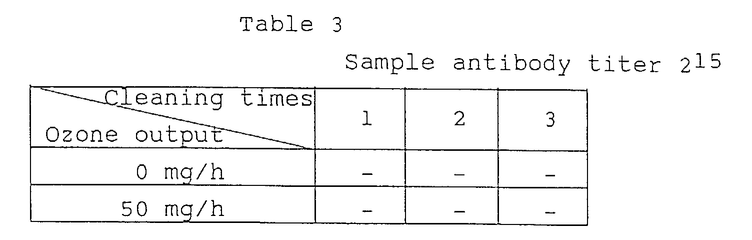

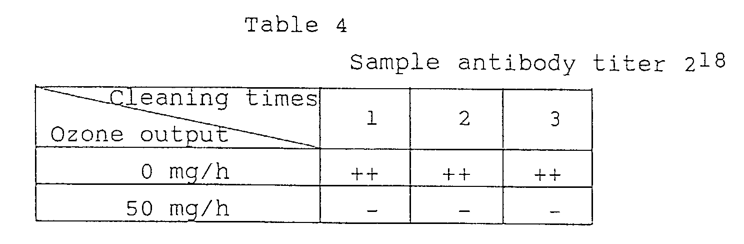

As shown in Tables 3, 4, without ozone treatment, although there was no carry-over in the sample of antibody titer of 2¹⁵, the cleaning was insufficient and carry-over was found in the sample with the antibody titer of 2¹⁸. On the contrary, with ozone treatment, in both samples of titer 2¹⁵ and 2¹⁸, there was no carry-over at all, and excellent cleaning effects were noted.

### Example 3 (combination of Examples 1 and 2)

The embodiment shown in Fig. 4 represents an apparatus designed for combination between the cleaning by ozone-containing air same as in Example 1 and the cleaning by fine foams of ozone-containg air in water same as in Example 2.

In Fig. 4, numeral 46 denotes a sample nozzle, 47 is a sample cup, 48 is a discharge port, 49 is a cleaning vessel, 47' is a reaction vessel, 50 is a foam generator, 51 is an electromagnetic valve, 52 is a water feed port, 53 is a coupling part, 54 is an ozone generator, 55 is an air pump, 56 is a coupling part, 57 is an electromagnetic valve, 58 is a water feed pump, 59 is an electromagnetic valve, 60 is an electromagnetic valve, 61 is a coupling part, 62 is a syringe, 63 is a plunger, 64 is a feed water tank, 65 is a waste liquid tank, 66 is an ozone processing device, 67 is a filter, and 68 is a suction pump.

This embodiment includes both constitutions same as in the first embodiment (Example 1) and second embodiment (Example 2), and is capable of combining the cleaning by ozone-containing air and the cleaning by fine foams of ozone-containing air in water.

### Example 4

This embodiment relates to an apparatus composed for cleaning the devices with ozone-desolved solution.

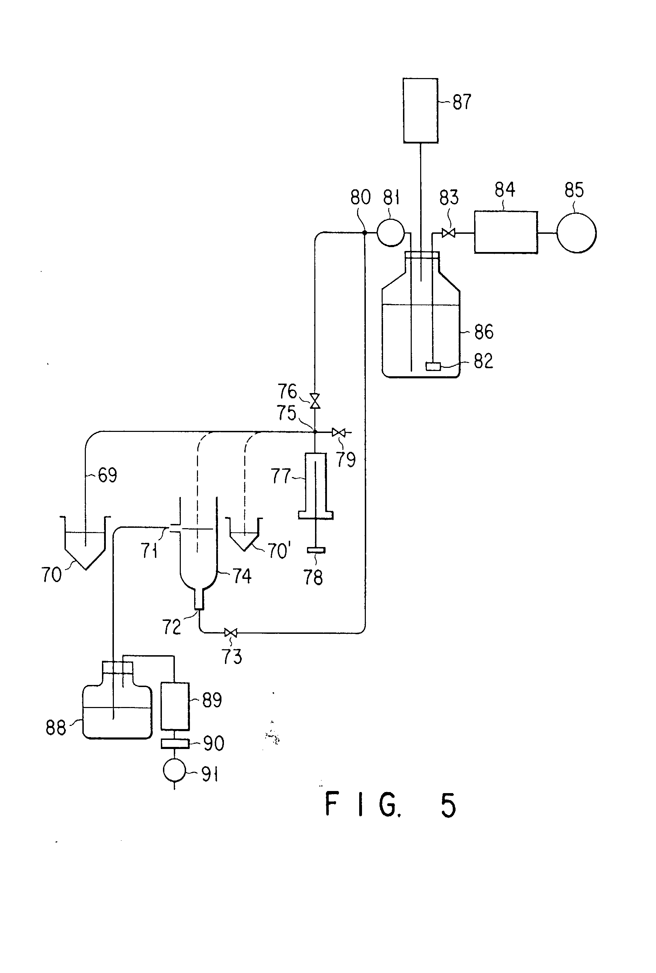

Fig. 5 shows an apparatus conforming to the third embodiment. In the drawing, numeral 69 denotes a sample nozzle, 70 is a sample cup, 70' is a reaction vessel, 71 is a discharge port, 72 is a feed water port, 73 is an electromagnetic valve, 74 is a cleaning vessel, 75 is a coupling part, 76 is an electromagnetic valve, 77 is a syringe, 78 is a plunger, 79 is an electromagnetic valve, 80 is a coupling part, 81 is a feed water pump, 82 is an ozone ventilation hole, 83 is an electromagnetic valve, 84 is an ozone generator, 85 is an air pump, 86 is a feed water tank, 87 is an ozone processing device, 88 is a waste liquid tank, 89 is an ozone processing device, 90 is a filter, and 91 is a suction pump.

This embodiment, as compared with the first and second embodiments, is poor in effects, but it can be applied in the case where it is difficult to mechanically ventilate the nozzle or container with ozone or ozone-containing air.

### <Measurement of cleaning effect〉

Sucking and discharging 25 µL of anti-HBs antibody solution, the nozzle 69 was cleaned by using the apparatus shown in Fig. 5. The antibody titer of the anti-HBs antibody solution was 2¹⁸, which was measured with anti-HBs antibody measuring reagent available from Chemical and Serum Therapy Research Institute under the trade name of Serocrit anti-HBs. The cleaning conditions were as follows.
· Liquid volume in vessel 74: Purified water 1.5 ml
· Capacity of feed water tank 86: 10 ml
· Ozone output of ozone generator 84: 50 mg/h
· Air feed rate of air pump 85: 1.2 L/min
· Cleaning liquid discharge volume: 200 µL

Same as in Examples 1, 2, after cleaning, the presence or absence of anti-HBs antibody remained in the nozzle 69 was investigated. The measuring operation was same as in Examples 1, 2.

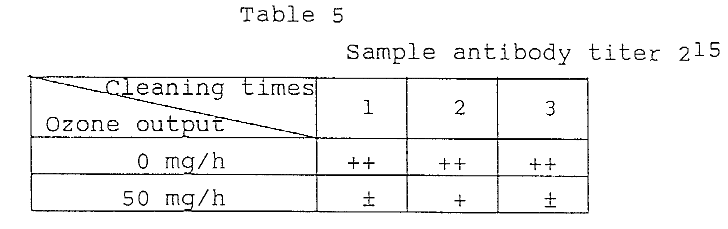

As shown in Table 5, the method of this embodiment is more effective than in the case without ozone treatment.

### Example 5

The invention may be applied not only in cleaning of sample nozzles as disclosed in Examples 1 to 4, but also in cleaning of various solid surfaces such as reaction vessels. This Example 5 is to experimentally prove the effect when used in cleaning of reaction vessels.

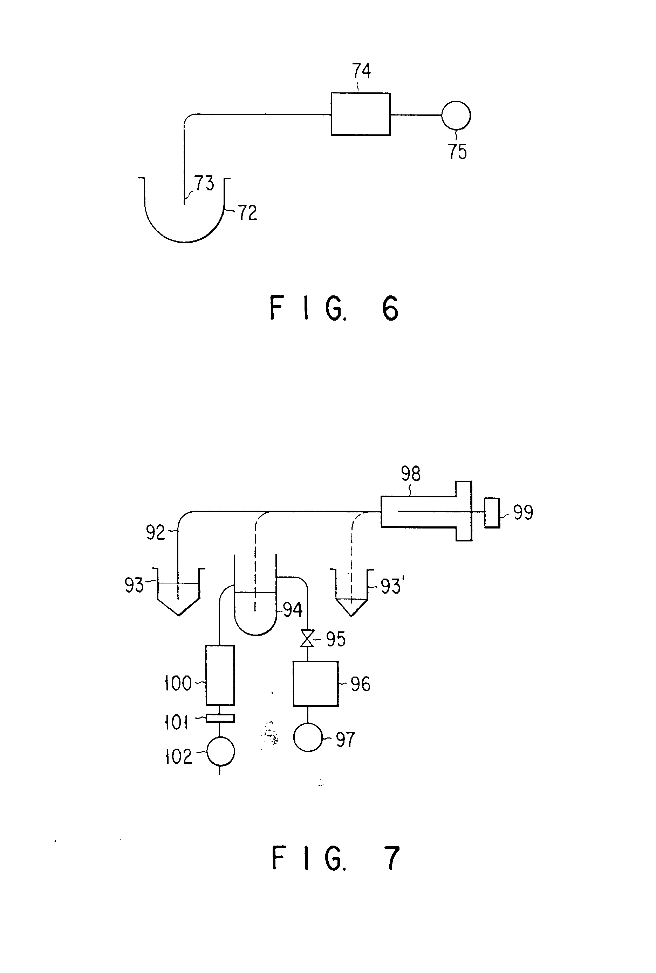

Fig. 6 is a diagram for explaining the method of this embodiment. In the diagram, numeral 72 denotes a microplate, 73 is an ozone blowing nozzle, 74 is an ozone generator, and 75 is an air pump.

In the following procedure, human IgG was dispensed in the microplate, and the human IgG was artificially adsorbed on the inner surface of the microplate, and the cleaning effect by ozone was investigated.
(1) Series of multiple diluted solutions of 10 µg/ml of human IgG were prepared by using 0.01M PBS pH 7.4. The solution was, by 50 µl each, dispensed into each well of module U-16 Maxisoap (tradename) which is a microplate available from NUNC, and was immobilized overnight at 4°C.
(2) The microplate was washed three times using 250 µl of 0.01M PBS pH 7.4 in each time the washing solution was shaken off.
(3) In the same manner as shown in Fig. 6, ozone-containing air was blow into the inner surface of the microplate. The conditions were as follows: ozone output of 50 mg/h, air blow rate of 1.2 L/min, blowing time of 5 sec and 60 sec.
(4) The cleaning effect by the ozone-containing air was evaluated from the reactivity of the anti-human IgG sensitized particles (Olympus Optical Co., Ltd.) with the inner surface of the microplate.

The results were as shown in Table 6. It was proved from the result that the invention is effective in cleaning of the human IgG adsorbed on the solid surface of the microplate, which is difficult to be cleaned mechanically due to the adsorption.

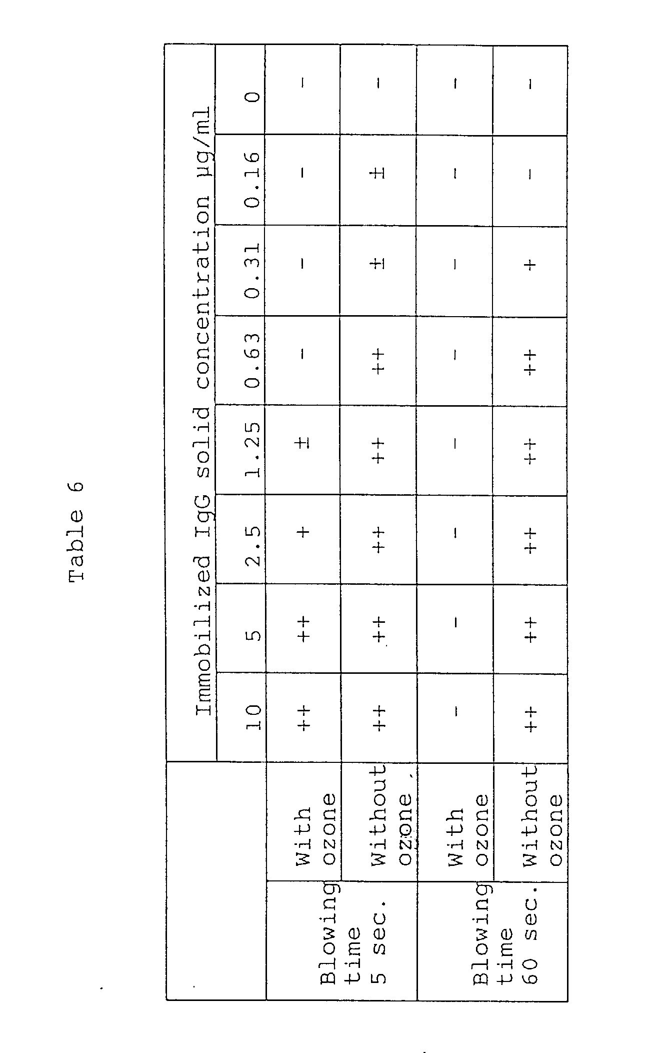

Note that the cleaning method of the embodiment may be realized not only by blowing the ozone-containing air as shown in Fig. 6, but also by using aqueous solution mixed with foams of ozone-containing air, ozone-dessolved aqueous solution, or the like. In the cleaning of the solid surface, however, it is more preferable to blow the ozone-containing gas in a dry state.

### Example 6

This embodiment relates to cleaning of enzyme.

According to the procedure below, in the similar manner shown in Fig. 6 and described in Example 5, peroxidase was artificially adsorbed on the inner surface of the microplate, and the cleaning effect by ozone was investigated.
(1) Series of multiple diluted solutions of 500 ng/ml of peroxidase were prepared by using 0.01 M PBS, pH 7.4. The solution was dispensed, by 100 µl each, into each well of flat bottom Maxisoap (tradename) which is a microplate available from NUNC. At room temperature (RT), reaction was performed for 1 hour, and the peroxidase was artificially adsorbed on the inner surface of the microplate.
(2) After washing the microplate five times using 200 µl of purified water in each time, the purified water was shaken off.
(3) Ozone-containing air was blown into each well. The conditions were as follows: ozone output of 0 mg/h, 50 mg/h, air blowing rate of 1.2 L/min, blowing time of 10 sec.
(4) 0.2M phosphate-citrate buffer, pH 4.5, H₂O₂ 0.04%, o-phenylene diamine 0.5 mg/ml were added to each well by 100 µl each. The reaction was allowed to cause for 15 min at room temperature for color developing.
(5) Adding 5N H₂SO₄ by 50 µl/well, the measurement was taken at OD 490 nm. The results were as shown in Table 7.

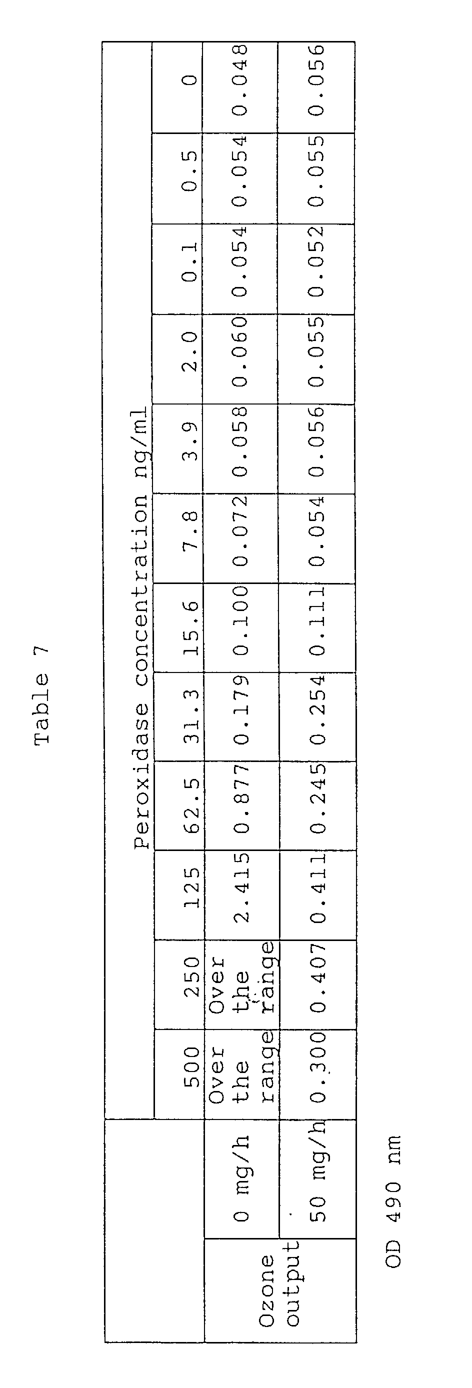

As shown in Table 7, since color developing was surpressed or deprived of by treatment with ozone, it is found that the activity of peroxidase was lowered. However, the activity lowering effect was not so significant as in the case of antibody and antigen in the foregoing embodiments. Anyway, it suggests that it is sufficiently applicable in cleaning of enzyme. The enzymes to which the invention is applicable are not limited to peroxidase alone, but the present inventions may be considered effective also on the labeling enzymes used in immunoassay or DNA assay such as alkaline phosphatase, glucose oxidase, luciferase, B-D-galactosidase and others.

### Example 7

This embodiment relates to an apparatus having the mechanism for cleaning the outer surface of the sample nozzle by blowing ozone-containing air to the sample nozzle.

Fig. 7 shows an apparatus conforming to this embodiment. In Fig. 7, numeral 92 is a sample nozzle, 93 is a sample cup, 93' is a reaction vessel, 94 is a cleaning vessel, 95 is an electromagnetic valve, 96 is an ozone generator, 97 is an air pump, 98 is a syringe, 99 is a plunger, 100 is an ozone processing device, 101 is a filter, and 102 is an suction pump.

In cleaning by using this apparatus, the air is sent into the ozonizer 96 by the air pump 97. Thus, the ozone-containing air is blown into the sample nozzle 92 in the cleaning vessel 94, thereby effectiving the cleaning of the sample nozzle 92.

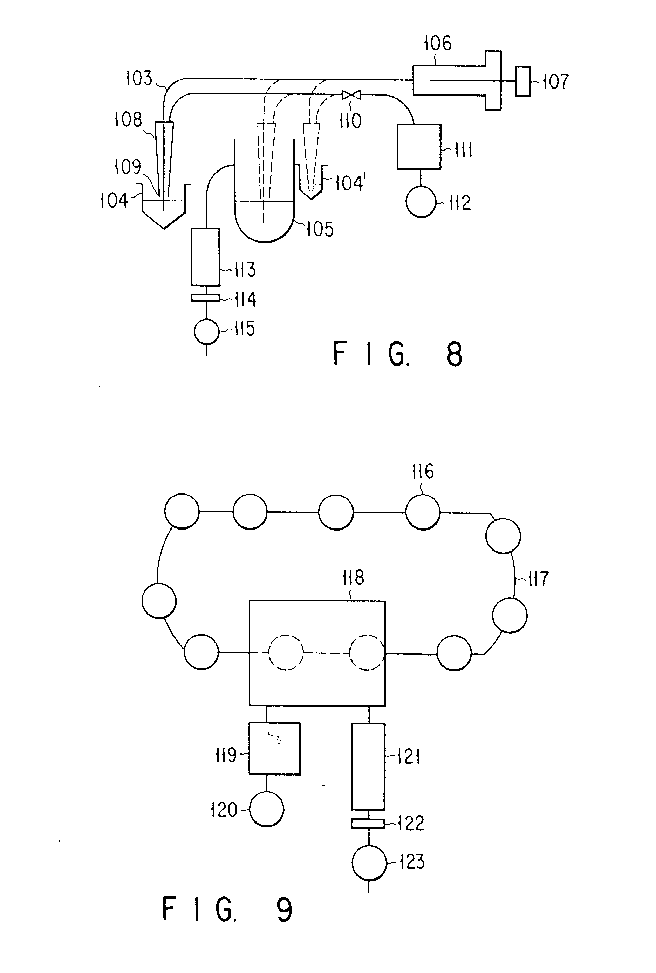

The same cleaning may be effected also by using the apparatus shown in Fig. 8. In Fig. 8, numeral 103 denotes a sample nozzle, 104 is a sample cup, 104' is a reaction vessel, 105 is a cleaning vessel, 106 is a syringe, 107 is a plunger, 108 is an ozone blowing nozzle, 109 is an ozone blowing port, 110 is an electromagnetic valve, 111 is an ozone generator, 112 is an air pump, 113 is an ozone processing device, 114 is a filter, and 115 is an suction pump.

In the embodiment in Fig. 8, air is sent into the ozonizer 111 by the air pump 112. The ozone-containing air produced in the ozonizer 111 is blown to the outer surface of the sample nozzle 103 from the ozone blowing port through the ozone blowing nozzle 108, inside the cleaning vessel 105. As a result, the outer surface of the sample nozzle 103 is cleaned.

The cleaning by the apparatus shown in Fig. 7 and Fig. 8 is also effective for blowing out the water drops left over on the front end of the sample nozzle after sample dispensing or washing.

Note that the apparatus in Fig. 7 and Fig. 8 may be used also in combination with the apparatus of Examples 1 to 4.

### Example 8

The embodiment relates to an apparatus arranged for cleaning a reaction vessel by passing the vessel in a chamber filled with ozone-containing air.

Fig. 9 shows the apparatus used in the embodiment. In the drawing, numeral 116 is a reaction vessel, 117 is an endless belt for conveying reaction vessel, 118 is an ozone treating chamber, 119 is an ozone generator, 120 is an air pump, 121 is an ozone processing device, 122 is a filter, and 123 is a suction pump. In addition, near the inlet and outlet of the ozone treating chamber 118, air curtain or similar shielding means may be provided in order to prevent leak of ozone gas, or the treating chamber is set at a slightly negative pressure as compared with the atmosphere. In the apparatus, air is sent into the ozonizer 119 by the air pump 120, and the ozone treating chamber 118 is filled with ozone-containing air. The reaction vessel 116 which is finished in sample treatment by dispensing means and measuring means (not shown herein) and finished in discharge of liquid by the liquid discharge means (not shown herein) is traveled by pulling the endless belt 117 for conveying the reaction vessel, and is passed through the ozone treating chamber 118 in order to be treated. Although not shown in the drawing, convection generating means such as fan is provided in the ozone treating chamber 118, and the ozone flow is blown to the container introduced into the treating chamber. The ozone concentration in the treating chamber is also controlled.

In this embodiment, ozone treatment is carried out after discharging the liquid in the reaction vessel. But only ozone treatment may be carried out and liquid discharge may be omitted, where the liquid volume is very small, or the liquid depth is very shallow or when analysis element of poor wetting is used.

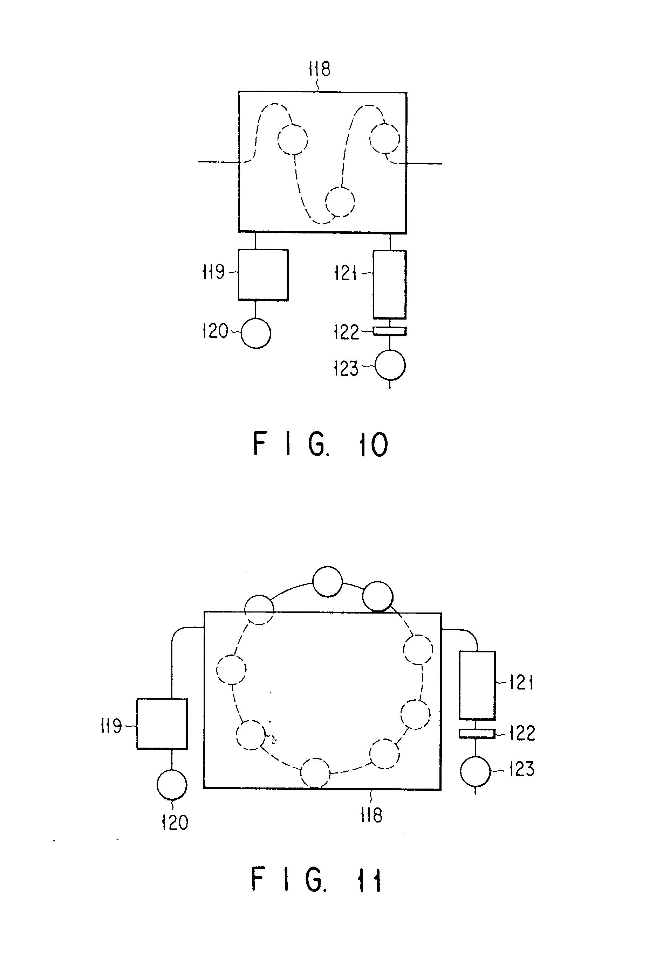

To extend the treating time, as shown in Fig. 10, the reaction vessel may be moved in a zigzag form in the ozone treating chamber. Or as shown in Fig. 11, it may be also composed to stay the reaction vessels within the ozone treating chamber 118 except for reaction operation.

The above mentioned cleaning mechanism may be also effectively applied to the reaction vessel which is transferred on an endless reaction line.

In addition, water cleaning apparatus of the reaction vessel may be also provided before and after the ozone treating chamber 118 or within the treating chamber 118.

The invention may be modified in various way, not limited to the illustrated embodiments. For example, when the inner wall of the nozzle is cleaned with ozone gas, the nozzle may be introudced into the treating chamber as in the eighth embodiment, while the gas pressure in the treating chamber may be changed by increasing or decreasing for blowing the ozone gas from outside into the chamber.

Furthermore, if the tip of the sample nozzle is exchangeable, it is possible to remove the tip and use the tip again after treatment according to the eighth embodiment.

As described in detail herein, the present invention is extremely effective for cleaning the reaction vessels of the analyzing apparatus, sample nozzles, and reagent dispensing nozzles which are used for handling or treating biological high polymers such as antigen, antibody, enzyme and DNA as samples or reagents, in particular. The sample nozzles or reagent despensing nozzles are not limitted to that of pipe-shaped, but the present invention is also applicable to the nozzles of various form, diluter and the like so far as the ozone-containing fluid can be contacted with necessary parts of the devices.

## Claims

1. A method of avoiding carry-over and/or contamination in handling or treating liquids as samples or reagents containing active substances selected from the group consisting of an antigen, antibody, DNA, RNA, receptor, complement, enzyme, and combinations thereof, in an apparatus successively applying a biological activity measurement to a plurality of said liquids, by using the same device for said measurement, comprising the steps of:
(a) bringing a surface of said device into contact with said liquids during at least one step of applying said measurement to said liquids;
(b) moving said liquids away from the surface of said device after application of said measurement of said liquids;
(c) subjecting said device to a cleaning operation;
(d) bringing said device into contact with said next liquids during at least one step of application of said next measurement of said next liquids;
characterized in that step (c) comprises
supplying an ozone gas onto the surface of said device, prior to bringing the surface of said device into contact with the next liquids, the contact time between said ozone gas and said device surface being less than one minute which is sufficient for substantially inactivating the biologically active substances in said liquids adsorbed on said device, so that said activity does not adversely affect the next measurement of said next liquids.

2. The method according to claim 1, wherein said biological activity measurement is an immunological measurement.

3. The method according to claim 1, wherein said ozone gas is in a dry state.

4. The method according to claim 1, wherein said ozone gas is used in the form of a mixture with another gas.

5. The method according to claim 1, wherein said ozone gas is supplied in the form of bubbles in a liquid.

6. The method according to claim 1, wherein said device is a dispensing nozzle for sucking and/or discharging said test sample or said reagent for the dispensing purpose.

7. The method according to claim 6, wherein said ozone gas is allowed to flow through a fluid passageway formed within said dispensing nozzle for a predetermined period of time.

8. The method according to claim 7, wherein said ozone gas supply in step (c) is performed into said dispensing nozzle during the period between completion of the discharge of a first sample or reagent from the dispensing nozzle and initiation of the suction of a second sample or reagent into the dispensing nozzle.

9. The method according to claim 1, wherein said device is a container for receiving said test sample and/or said reagent.

10. The method according to claim 9, wherein said device is a reaction vessel for carrying out a reaction between said test sample and said reagent.

11. The method according to claim 10, wherein an amount of an ozone gas is blown in step (c) against the inner surface of said reaction vessel, wherein said amount is sufficient for substantially inhibiting the biological activity of said liquids.

12. The method according to claim 10, wherein said ozone gas supply in step (c) is performed into said reaction vessel during the period between completion of the discharge of the reaction mixture between the first sample and the first reagent from the reaction vessel and initiation of the dispensing of the second sample and the second reagent into the reaction vessel.

13. The method according to claim 1, wherein the supply time of said ozone gas in step (c) is about 3 to 10 seconds.

14. The method according to claim 13, wherein said biological activity measurement is successively applied substantially continuously to a plurality of said liquids.

15. The method according to claim 1, further comprising the step of:
(e) washing the surface of said device with a washing water which does not contain a detergent for partly removing said liquids attached to the device surface, said step (e) being carried out between steps (b) and (c).

16. The method according to claim 15, further comprising the step of:
(f) supplying ozone gas into said washing water discharged in said washing step, said washing water containing said liquids, so that the activity of said liquids contained in the washing water, does not adversely affect the next measurement.

17. The method according to claim 16, wherein the liquid material discharged in step (f) is used in step (e) in respect of the device used for the measurement of said next liquids.

## Patentansprüche

1. Verfahren zur Vermeidung eines Verschleppens und/oder einer Kontamination beim Handhaben oder Behandeln von Flüssigkeiten als Proben oder Reagenzien, welche aktive Substanzen enthalten, die ausgewählt sind aus der Gruppe bestehend aus einem Antigen, Antikörper, DNA, RNA, Rezeptor, Komplement, Enzym und Kombinationen davon, in einem Gerät, das nacheinander mit einer Mehrzahl der Flüssigkeiten eine biologische Aktivitätsmessung durchführt, indem für die Messung dieselbe Vorrichtung verwendet wird, welches die folgenden Schritte umfaßt:
(a) In-Kontakt-Bringen einer Oberfläche der Vorrichtung mit den Flüssigkeiten während wenigstens eines Schrittes der Durchführung der Messung mit den Flüssigkeiten;
(b) Wegbewegen der Flüssigkeiten von der Oberfläche der Vorrichtung nach der Durchführung der Messung mit den Flüssigkeiten;
(c) Unterwerfen der Vorrichtung unter einen Reinigungsvorgang;
(d) In-Kontakt-Bringen der Vorrichtung mit den nächsten Flüssigkeiten während wenigstens eines Schrittes der Durchführung der nächsten Messung mit den nächsten Flüssigkeiten;
dadurch gekennzeichnet, daß der Schritt (c) umfaßt:
Zuführen von Ozongas auf die Oberfläche der Vorrichtung, bevor die Oberfläche der Vorrichtung mit den nächsten Flüssigkeiten in Kontakt gebracht wird, wobei die Kontaktzeit zwischen dem Ozongas und der Vorrichtungsoberfläche weniger als eine Minute beträgt, was ausreicht, um die biologisch aktiven Substanzen in den Flüssigkeiten, die auf der Vorrichtung adsorbiert sind, im wesentlichen zu inaktivieren, so daß sich die Aktivität nicht nachteilig auf die nächste Messung der nächsten Flüssigkeiten auswirkt.

2. Das Verfahren gemäß Anspruch 1, wobei die biologische Aktivitätsmessung eine immunologische Messung ist.

3. Das Verfahren gemäß Anspruch 1, wobei das Ozongas in einem trockenen Zustand ist.

4. Das Verfahren gemäß Anspruch 1, wobei das Ozongas in der Form einer Mischung mit einem anderen Gas verwendet wird.

5. Das Verfahren gemäß Anspruch 1, wobei das Ozongas in der Form von Blasen in einer Flüssigkeit zugeführt wird.

6. Das Verfahren gemäß Anspruch 1, wobei die Vorrichtung eine Ausgabeöffnung zum Ansaugen und/oder Abgeben der Testprobe oder des Reagenzes zu Zwecken der Ausgabe ist.

7. Das Verfahren gemäß Anspruch 6, wobei das Ozongas für eine vorherbestimmte Zeitperiode durch einen Fluiddurchgang, der innerhalb der Ausgabeöffnung ausgebildet ist, fließen gelassen wird.

8. Das Verfahren gemäß Anspruch 7, wobei die Ozongaszufuhr in die Ausgabeöffnung in Schritt (c) während der Periode zwischen dem Abschluß der Abgabe einer ersten Probe oder eines Reagenzes aus der Ausgabeöffnung und dem Beginn des Ansaugens einer zweiten Probe oder eines Reagenzes in die Ausgabeöffnung durchgeführt wird.

9. Das Verfahren gemäß Anspruch 1, wobei die Vorrichtung ein Behälter ist, um die Testprobe und/oder das Reagenz aufzunehmen.

10. Das Verfahren gemäß Anspruch 9, wobei die Vorrichtung ein Reaktionsgefäß ist, um eine Reaktion zwischen der Testprobe und dem Reagenz durchzuführen.

11. Das Verfahren gemäß Anspruch 10, wobei eine Menge an Ozongas in Schritt (c) gegen die innere Oberfläche des Reaktionsgefäßes geblasen wird, wobei die Menge ausreicht, um die biologische Aktivität der Flüssigkeiten im wesentlichen zu inhibieren.

12. Das Verfahren gemäß Anspruch 10, wobei die Ozongaszufuhr in das Reaktionsgefäß in Schritt (c) während der Periode zwischen der Beendigung der Abgabe der Reaktionsmischung zwischen der ersten Probe und dem ersten Reagenz aus dem Reaktionsgefäß und dem Beginn der Abgabe der zweiten Probe und des zweiten Reagenzes in das Reaktionsgefäß durchgeführt wird.

13. Das Verfahren gemäß Anspruch 1, wobei die Zuführzeit des Ozongases in Schritt (c) ca. 3 bis 10 Sekunden beträgt.

14. Das Verfahren gemäß Anspruch 13, wobei die biologische Aktivitätsmessung nacheinander im wesentlichen kontinuierlich mit einer Mehrzahl der Flüssigkeiten durchgeführt wird.

15. Das Verfahren gemäß Anspruch 1, welches weiterhin den folgenden Schritt umfaßt:
(e) Waschen der Oberfläche der Vorrichtung mit einem Waschwasser, welches kein Detergens enthält, um die Flüssigkeiten, die an der Oberfläche der Vorrichtung haften, teilweise zu entfernen, wobei der Schritt (e) zwischen den Schritten (b) und (c) durchgeführt wird.

16. Das Verfahren gemäß Anspruch 15, welches weiterhin den folgenden Schritt umfaßt:
(f) Zuführen von Ozongas in das Waschwasser, welches in dem Waschschritt abgegeben wird, wobei das Waschwasser die Flüssigkeiten enthält, so daß sich die Aktivität der Flüssigkeiten, die in dem Waschwasser enthalten sind, nicht nachteilig auf die nächste Messung auswirkt.

17. Das Verfahren gemäß Anspruch 16, wobei das flüssige Material, welches in Schritt (f) abgegeben wird, in Bezug auf die Vorrichtung, die zur Messung der nächsten Flüssigkeiten verwendet wird, in Schritt (e) verwendet wird.

## Revendications

1. Procédé pour éviter un transfert et/ou une contamination dans la manipulation pour le traitement de liquides en tant qu'échantillons ou réactifs contenant des substances actives choisies dans le groupe formé par un antigène, un anticorps, un ADN, un ARN, un récepteur, un complément, une enzyme et leurs combinaisons, dans un appareil mettant en oeuvre successivement une mesure d'activité biologique à une pluralité desdits liquides, en utilisant le même dispositif pour ladite mesure, comprenant les étapes consistant:
(a) à amener une surface dudit dispositif en contact avec lesdits liquides pendant au moins une étape de mise en oeuvre de ladite mesure auxdits liquides;
(b) à éloigner lesdits liquides de la surface dudit dispositif après mise en oeuvre de ladite mesure desdits liquides;
(c) à soumettre ledit dispositif à une opération de nettoyage;
(d) à amener ledit dispositif en contact avec lesdits liquides suivants pendant au moins une étape de mise en oeuvre de la mesure suivante desdits liquides suivants;
caractérisé en ce que l'étape (c) consiste
à fournir un gaz à base d'ozone sur la surface dudit dispositif, avant d'amener la surface dudit dispositif en contact avec les liquides suivants, la durée de contact entre ledit gaz à base d'ozone et ladite surface de dispositif étant inférieure à une minute qui suffit à inactiver pour l'essentiel les substances biologiquement actives dans lesdits liquides adsorbés sur ledit dispositif, de sorte que ladite activité n'affecte pas de manière défavorable ladite mesure suivante desdits liquides sui-vants.

2. Procédé selon la revendication 1, dans lequel ladite mesure d'activité biologique est une mesure immunologique.

3. Procédé selon la revendication 1, dans lequel ledit gaz à base d'ozone se trouve à l'état sec.

4. Procédé selon la revendication 1, dans lequel ledit gaz à base d'ozone est utilisé sous forme d'un mélange avec un autre gaz.

5. Procédé selon la revendication 1, dans lequel ledit gaz à base d'ozone est fourni sous forme de bulles dans un liquide.

6. Procédé selon la revendication 1, dans lequel ledit dispositif constitue une buse de distribution pour aspirer et/ou décharger ledit échantillon de test ou ledit réactif à des fins de distribution.

7. Procédé selon la revendication 6, dans lequel ledit gaz à base d'ozone peut s'écouler à travers un passage de fluide formé à l'intérieur de ladite buse de distribution pendant une durée prédéterminée.

8. Procédé selon la revendication 7, dans lequel ladite alimentation en gaz à base d'ozone dans l'étape (c) est réalisée dans ladite buse de distribution pendant l'intervalle de temps compris entre l'achèvement de la décharge d'un premier échantillon ou réactif à partir de la buse de distribution et l'amorçage de l'aspiration d'un deuxième échantillon ou réactif dans la buse de distribution.

9. Procédé selon la revendication 1, dans lequel ledit dispositif est un récipient pour recevoir ledit échantillon de test et/ou ledit réactif.

10. Procédé selon la revendication 9, dans lequel ledit dispositif est un récipient de réaction destiné à réaliser une réaction entre ledit échantillon de test et ledit réactif.

11. Procédé selon la revendication 10, dans lequel une certaine quantité d'un gaz à base d'ozone est soufflée à l'étape (c) contre la surface interne dudit récipient de réaction, dans lequel ladite quantité suffit à inhiber pour l'essentiel l'activité biologique desdits liquides.

12. Procédé selon la revendication 10, dans lequel ladite alimentation en gaz à base d'ozone à l'étape (c) est réalisée dans ledit récipient de réaction pendant l'intervalle de temps compris entre l'achèvement de la décharge du mélange réactionnel entre le premier échantillon et le premier réactif à partir du récipient de réaction, et l'amorçage de la distribution du deuxième échantillon et du deuxième réactif dans le récipient de réaction.

13. Procédé selon la revendication 1, dans lequel la durée d'alimentation en gaz à base d'ozone à l'étape (c) est d'environ 3 à 10 secondes.

14. Procédé selon la revendication 13, dans lequel ladite mesure d'activité biologique est appliquée successivement pour l'essentiel en continu à une pluralité desdits liquides.

15. Procédé selon la revendication 1, comprenant de plus l'étape consistant:
(e) à laver la surface dudit dispositif avec une eau de lavage qui ne contient pas de détergent en vue de retirer partiellement lesdits liquides fixés à la surface du dispositif, ladite étape (e) étant effectuée entre les étapes (b) et (c).

16. Procédé selon la revendication 15, comprenant de plus l'étape consistant:
(f) à introduire le gaz à base d'ozone dans ladite eau de lavage déchargée dans ladite étape de lavage, ladite eau de lavage contenant lesdits liquides, de sorte que l'activité desdits liquides contenus dans l'eau de lavage n'affecte pas de manière défavorable la mesure suivante.

17. Procédé selon la revendication 16, dans lequel la matière liquide déchargée dans l'étape (f) est utilisée dans l'étape (e) en ce qui concerne le dispositif utilisé pour la mesure desdits liquides suivants.
